# EUROPEAN PATENT APPLICATION

(11) **EP 3 175 713 A1**
(43) Date of publication of application: **07.06.2017**
(21) Application number: 15827775.6
(22) Date of filing: 30.07.2015
(51) Int. Cl.: A01N 63/00, A01N 25/02, A01N 25/22, A01P 1/00, A23L 3/3571, A61K 9/10, A61K 9/12, A61K 35/54, A61K 38/46, A61K 47/04, A61K 47/10, A61P 31/04, A61P 31/12, A61P 31/14, C12N 9/36

(54) **WATER-CONTAINING FLUID COMPOSITION, METHOD FOR PRODUCING SAME, PROCESSED LYSOZYME AND/OR SALT THEREOF, AND METHOD FOR PRODUCING SAME**

(30) Priority: 31.07.2014 JP 2014157145
(71) Applicant: Kewpie Corporation, Tokyo 150-0002 (JP)
(72) Inventor: SUGISAKA, Arisa, Chofu-shi Tokyo 182-0002 (JP); YODA, Shoichi, Chofu-shi Tokyo 182-0002 (JP); SASAHARA, Ryo, Chofu-shi Tokyo 182-0002 (JP); SATO, Miki, Chofu-shi Tokyo 182-0002 (JP); TAKAHASHI, Hajime, Tokyo 108-8477 (JP)
(74) Representative: Winter, Brandl, Fürniss, Hübner, Röss, Kaiser, Polte - Partnerschaft mbB
(86) International application number: PCT/JP2015/071726
(87) International publication number: WO 2016/017784

(57) **Abstract**

Provided is a water-containing fluid composition comprising a processed lysozyme and/or a salt thereof, the water-containing fluid composition having a fluorescence intensity specified below of 5000 or more, wherein the processed lysozyme and/or the salt thereof is dissolved in the water-containing fluid composition while being in contact with a polar organic solvent, and a content of the polar organic solvent in the water-containing fluid composition is 55 mass% or more and 90 mass% or less. The fluorescence intensity: a fluorescence intensity measured under conditions of an excitation wavelength of 390 nm (excitation bandwidth of 10 nm) and a fluorescence wavelength of 470 nm (fluorescence bandwidth of 10 nm), for a solution obtained by adding 25 µL of an 8 mM solution of 1,8-anilinonaphthalenesulfonic acid in methanol to 5 mL of a diluted solution obtained by diluting the water-containing fluid composition with a phosphate buffer solution (pH 7.0) to a concentration in terms of solid content of the processed lysozyme and/or the salt thereof of 0.05 mass% and a concentration of phosphate of 0.2 M, and thereafter allowing the solution to react at room temperature for 30 minutes.

## Description

### Technical Field

The present invention relates to a water-containing fluid composition, a method for producing the same, a processed lysozyme and/or a salt thereof, and a method for producing the same.

### Background Art

Lysozymes are enzymes that are widely present in the living world such as eggs, tissues and body fluids of animals, and plants. Industrially, lysozymes isolated from egg whites are applied to foods and pharmaceutical products (Patent Literature 1). For example, lysozymes are widely used as disinfectant components because of their antibacterial action.

### Citation List

### Patent Literature

Patent Literature 1: Japanese Unexamined Patent Publication No. H5-221875

### Summary of Invention

### Problems to be Solved by the Invention

The inventors have found that a processed lysozyme obtained by modifying a lysozyme under specific conditions has a norovirus inactivating action, and a reduction in the norovirus inactivating action can be suppressed by storing the processed lysozyme in the presence of a polar organic solvent, thereby arriving at the present invention.

The present invention provides a water-containing fluid composition that can suppress a reduction in the norovirus, inactivating action, a method for producing the same, a processed lysozyme and/or a salt thereof, and a method for producing the same.

### Means for Solving the Problems

1. A water-containing fluid composition according to one aspect of the present invention comprises a processed lysozyme and/or a salt thereof, the water-containing fluid composition having a fluorescence intensity specified below of 5000 or more, wherein the processed lysozyme and/or the salt thereof is dissolved in the water-containing fluid composition while being in contact with a polar organic solvent, and a content of the polar organic solvent in the water-containing fluid composition is 55 mass% or more and 90 mass% or less.
   The fluorescence intensity: a fluorescence intensity measured under conditions of an excitation wavelength of 390 nm (excitation bandwidth of 10 nm) and a fluorescence wavelength of 470 nm (fluorescence bandwidth of 10 nm), for a solution obtained by adding 25 µL of an 8 mM solution of 1,8-anilinonaphthalenesulfonic acid in methanol to 5 mL of a diluted solution obtained by diluting the water-containing fluid composition with a phosphate buffer solution (pH 7.0) to a concentration in terms of solid content of the processed lysozyme and/or the salt thereof of 0.05 mass% and a concentration of phosphate of 0.2 M, and thereafter allowing the solution to react at room temperature for 30 minutes.
2. In the water-containing fluid composition according to 1 above, an anti-norovirus activity specified below can be 3.0 or more. The anti-norovirus activity: a value obtained by subtracting a logarithm of infectivity of a norovirus mixed solution obtained by mixing 1 part by mass of the water-containing fluid composition with respect to 0.1 parts by mass of a norovirus solution after being allowed to stand at room temperature for one minute, from a logarithm of infectivity of the norovirus mixed solution before being allowed to stand.
3. In the water-containing fluid composition according to 1 or 2 above, the fluorescence intensity can be 6000 or more.
4. In the water-containing fluid composition according to any one of 1 to 3 above, the polar organic solvent can contain ethanol, and a concentration of the ethanol can be 55 mass% or more and 90 mass% or less.
5. In the water-containing fluid composition according to any one of 1 to 4 above, a concentration in terms of solid content of the processed lysozyme and/or the salt thereof can be 0.1 mass% or more and 10 mass% or less.
6. The water-containing fluid composition according to any one of 1 to 5 above can further contain a water-soluble alcohol other than ethanol.
7. In the water-containing fluid composition according to any one of 1 to 6 above, the processed lysozyme and/or the salt thereof can be derived from an egg white lysozyme.
8. The water-containing fluid composition according to any one of 1 to 7 above can be packed into a spray container.
9. The water-containing fluid composition according to any one of 1 to 7 above can be packed into a pump container.
10. The water-containing fluid composition according to any one of 1 to 7 above can impregnate a non-woven fabric.
11. A method for inactivating bacteria and/or viruses according to one aspect of the present invention comprises: a step of bringing the water-containing fluid composition according to any one of 1 to 7 above into contact with a target.
12. A processed lysozyme and/or a salt thereof according to one aspect of the present invention has a fluorescence intensity, specified by the following definition, of 4000 or more:
   the fluorescence intensity: a fluorescence intensity measured under conditions of an excitation wavelength of 390 nm (excitation bandwidth of 10 nm) and a fluorescence wavelength of 470 nm (fluorescence bandwidth of 10 nm), for a solution obtained by adding 25 µL of an 8 mM solution of 1,8-anilinonaphthalenesulfonic acid in methanol to 5 mL of a diluted solution obtained by diluting with a phosphate buffer solution (pH 7.0) to a concentration in terms of solid content of the processed lysozyme and/or the salt thereof of 0.05 mass% and a concentration of phosphate of 0.2 M, and thereafter allowing the solution to react at room temperature for 30 minutes.
13. In the processed lysozyme and/or the salt thereof according to 12 above, an anti-norovirus activity specified below can be 2.0 or more.
   The anti-norovirus activity: a value obtained by subtracting a logarithm of infectivity of a norovirus mixed solution obtained by mixing a norovirus solution with a 2 mass% aqueous solution of the processed lysozyme and/or the salt thereof in equal amounts after the norovirus mixed solution is allowed to stand at room temperature for one minute, from a logarithm of infectivity of the norovirus mixed solution before being allowed to stand.
14. A method for producing the processed lysozyme and/or the salt thereof according to one aspect of the present invention is a method for producing the processed lysozyme and/or the salt thereof according to 12 or 13 above comprising: a first heating step of heating an aqueous solution of the lysozyme and/or the salt thereof having a light transmittance at 660-nm wavelength of more than 70%, a pH of 5.0 or more and 7.0 or less, and a concentration in terms of solid content of the lysozyme and/or the salt thereof of 0.5 mass% or more and 7 mass% or less, until the light transmittance at 660-nm wavelength of the aqueous solution reaches 70%; a second heating step of heating the aqueous solution after the first heating step until the light transmittance at 660-nm wavelength of the aqueous solution reaches a minimum value of less than 70% and thereafter heating the aqueous solution up to 70%; and a third heating step of further heating the aqueous solution after the second heating step in a state where the light transmittance at 660-nm wavelength of the aqueous solution is more than 70%.
15. In the method for producing the processed lysozyme and/or the salt thereof according to 14 above, the heating conditions in the third heating step can be such that the heating is performed until the light transmittance at 660-nm wavelength of a mixture of a filtrate obtained by filtering the aqueous solution obtained in the third heating step through a 0.45 µm membrane filter with ethanol at a mass ratio of 1:1 reaches 85% or more.
16. The method for producing the processed lysozyme and/or the salt thereof according to 14 or 15 above further comprises: a step of obtaining the processed lysozyme and/or the salt thereof in powder form by spray drying or freeze drying the aqueous solution after the third heating step.
17. A method for producing a water-containing fluid composition according to one aspect of the present invention comprises: a step of obtaining a water-containing fluid composition having a concentration of a polar organic solvent of 55 mass% or more and 90 mass% or less by mixing the processed lysozyme and/or the salt thereof obtained by the method according to any one of 14 to 16 above with the polar organic solvent.
18. A method for producing a water-containing fluid composition according to one aspect of the present invention comprises: a step of obtaining a water-containing fluid composition having a concentration of the polar organic solvent of 55 mass% or more and 90 mass% or less by mixing the processed lysozyme and/or salt thereof according to 12 or 13 above with the polar organic solvent.
19. In the method for producing a water-containing fluid composition according to 17 or 18 above, in the step of obtaining the water-containing fluid composition, the processed lysozyme and/or the salt thereof can be mixed with the polar organic solvent so that a concentration in terms of solid content of the processed lysozyme and/or the salt thereof in the water-containing fluid composition is 0.1 mass% or more 10 mass% or less.

### Effects of the Invention

The water-containing fluid composition according to any one of 1 to 10 above comprises the processed lysozyme and/or the salt thereof, and the water-containing fluid composition has a fluorescence intensity specified below of 5000 or more, wherein the processed lysozyme and/or the salt thereof is dissolved in the water-containing fluid composition while being in contact with the polar organic solvent, and the content of the polar organic solvent in the water-containing fluid composition is 55 mass% or more and 90 mass% or less, thereby having a norovirus inactivating action and being capable of maintaining the norovirus inactivating action over a long period of time (for example, for 2 weeks or more, generally 24 weeks or less, at 40°C).

The aforementioned water-containing fluid composition can be used, for example, for pharmaceutical products, quasi drugs, cosmetics, food washing applications, or sanitary applications. In this case, the aforementioned water-containing fluid composition can be used for norovirus removing applications. More specifically, the aforementioned water-containing fluid composition can be used, for example, in the form of being contained in a container (such as a container to which a nozzle, a spray, or the like is attached, or a container without such an attachment), or in the form of impregnating a non-woven fabric.

Further, the processed lysozyme and/or the salt thereof according to 12 or 13 above has a fluorescence intensity, as specified by the aforementioned definition, of 4000 or more, thereby having a norovirus inactivating action and being capable of maintaining the norovirus inactivating action over a long period of time by being brought into contact with the polar organic solvent.

Further, according to the method for producing the processed lysozyme and/or the salt thereof according to 14 to 16 above, the processed lysozyme and/or the salt thereof having a norovirus inactivating action and being capable of maintaining the norovirus inactivating action over a long period of time by being brought into contact with the polar organic solvent can be obtained by the first heating step, the second heating step, and the third heating step.

Further, according to the method for producing a water-containing fluid composition according to 17 and 19 above, the water-containing fluid composition having a norovirus inactivating action and being capable of maintaining the norovirus inactivating action over a long period of time can be obtained by including the step of obtaining a water-containing fluid composition having a concentration of a polar organic solvent of 55 mass% or more and 90 mass% or less by mixing the processed lysozyme and/or the salt thereof obtained by the method according to any one of 14 to 16 above with the polar organic solvent.

Further, according to the method for producing a water-containing fluid composition according to 18 or 19 above, the water-containing fluid composition having a norovirus inactivating action and being capable of maintaining the norovirus inactivating action over a long period of time can be obtained by including the step of obtaining a water-containing fluid composition having a concentration of a polar organic solvent of 55 mass% or more and 90 mass% or less by mixing the processed lysozyme and/or the salt thereof according to 12 or 13 above with the polar organic solvent.

### Brief Description of Drawings

[Figure 1] Figure 1 is a graph schematically showing a relationship between a heating time in a first heating step, a second heating step, and a third heating step, and a transmittance in a method for producing a processed lysozyme and/or a salt thereof according to one embodiment of the present invention.
[Figure 2] Figure 2 is an image showing the results of electrophoresis (SDS-PAGE) of a processed lysozyme and/or a salt thereof obtained in one example of the present invention.

### Embodiments for Carrying Out the Invention

Hereinafter, the present invention will be described in detail. In the present invention, "part(s)" means "part(s) by mass", and "%" means mass%, unless otherwise specified.

### <Water-containing fluid composition>

A water-containing fluid composition according to one embodiment of the present invention is a water-containing fluid composition comprising the processed lysozyme and/or the salt thereof, the water-containing fluid composition having a fluorescence intensity specified below of 5000 or more, wherein the processed lysozyme and/or the salt thereof is dissolved in the water-containing fluid composition while being in contact with a polar organic solvent, and the content of the polar organic solvent in the water-containing fluid composition is 55 mass% or more and 90 mass% or less.

The fluorescence intensity: a fluorescence intensity measured under conditions of an excitation wavelength of 390 nm (excitation bandwidth of 10 nm) and a fluorescence wavelength of 470 nm (fluorescence bandwidth of 10 nm), for a solution obtained by adding 25 µL of an 8 mM solution of 1,8-anilinonaphthalenesulfonic acid in methanol to 5 µL of a diluted solution obtained by diluting the water-containing fluid composition with a phosphate buffer solution (pH 7.0) to a concentration in terms of solid content of the processed lysozyme and/or the salt thereof of 0.05 mass% and a concentration of the phosphate of 0.2 M, and thereafter allowing the solution to react at room temperature for 30 minutes.

In the present invention, the "water-containing fluid composition" is a composition containing water and having a viscosity that allows deformation (for example, 1 to 100000 mPa·s), and is a concept including a liquid composition having a low viscosity that allows spraying and a gel-like composition. The solvent is not specifically limited as long as the processed lysozyme and/or the salt thereof is uniformly dissolved or dispersed therein, but water or alcohol can be used, for example. Further, in the present invention, the "room temperature" is 20°C or more and 25°C or less.

### [Fluorescence intensity and norovirus inactivating action]

For higher surface hydrophobicity, the fluorescence intensity of the water-containing fluid composition according to this embodiment, as specified by the aforementioned definition, is preferably 6000 or more, preferably 6500 or more, further preferably 7000 or more, and is generally 10000 or less. The fluorescence intensity of the water-containing fluid composition specified in the present invention is an index of surface hydrophobicity of proteins included in the water-containing fluid composition. That is, the higher the fluorescence intensity of the water-containing fluid composition specified in the present invention, the higher the surface hydrophobicity of proteins included in the water-containing fluid composition, and the higher the surface hydrophobicity of the water-containing fluid composition, the higher the norovirus inactivating action.

Therefore, the fluorescence intensity of the water-containing fluid composition according to this embodiment is mainly derived from the surface hydrophobicity of the processed lysozyme and/or the salt thereof contained in the water-containing fluid composition. Generally, when the surface hydrophobicity of proteins is increased, the hydrophobic portions of the proteins pull each other and aggregate together. The inventors have found that the higher the surface hydrophobicity of the processed lysozymes and/or the salt thereof, the norovirus inactivating action tends to be higher. That is, the processed lysozyme and/or the salt thereof according to this embodiment has a feature of having excellent norovirus inactivating action.

Although the reasons why the processed lysozyme and/or the salt thereof contained in the water-containing fluid composition according to this embodiment has excellent norovirus inactivating action are not clear, it is inferred that, in the first, the processed lysozymes and/or the salt thereof having surface hydrophobicity is easily bound to hydrophobic sites of norovirus, in the second, the processed lysozyme and/or the salt thereof is a lysozyme modified product, the processed lysozyme includes thiol groups (-SH) obtained by cleavage of at least some of S-S bonds contained in the lysozyme due to modification, and the thiol groups are bound to S-S bonds present on the surface of norovirus, and in the third, the processed lysozyme and/or the salt thereof has a three-dimensional structure that is easily bound to norovirus.

Further, although the reasons why the water-containing fluid composition according to this embodiment can suppress the reduction in the norovirus inactivating action are not clear, it is inferred that, since the processed lysozyme and/or the salt thereof is dissolved in the water-containing fluid composition according to this embodiment, thereby allowing the polar organic solvent contained in the water-containing fluid composition and the processed lysozyme and/or the salt thereof to be in contact with each other, the processed lysozyme and/or the salt thereof can maintain a three-dimensional structure that can exert the norovirus inactivating action.

That is, it is inferred that, since the processed lysozyme and/or the salt thereof has high surface hydrophobicity and therefore has high affinity with the polar organic solvent (particularly ethanol), the processed lysozyme and/or the salt thereof can maintain a three-dimensional structure that can exert the norovirus inactivating action in the state where the processed lysozyme is in contact with the polar organic solvent in the water-containing fluid composition, as a result of which it can maintain the norovirus inactivating action.

In the present invention, the fluorescence intensity of the water-containing fluid composition and the processed lysozyme and/or the salt thereof, which will be described below, is a value measured by the method disclosed in Canadian Institute of Food Science and Technology, 1985, Vol. 18, No. 4, p. 290-295. The phosphate buffer solution used for measuring the fluorescence intensity of the processed lysozyme and/or the salt thereof in the present invention is prepared using sodium dihydrogen phosphate and disodium hydrogen phosphate. Further, the fluorescence intensity of the processed lysozyme and/or the salt thereof in the present invention is a value obtained by subtracting a blank value, which has been obtained by separately measuring the fluorescence intensity of a 0.2 M phosphate buffer solution (pH 7.0, containing sodium dihydrogen phosphate and disodium hydrogen phosphate as phosphates) as the blank value.

The fluorescence intensity of the water-containing fluid composition according to this embodiment and the processed lysozyme and/or the salt thereof according to the later-described embodiment is a value measured using a fluorescent spectrophotometer, FP-8500, manufactured by JASCO Corporation, under conditions of an excitation wavelength of 390 nm, an excitation bandwidth of 10 nm, a fluorescence wavelength of 470 nm, a fluorescence bandwidth of 10 nm, a response of 0.5 sec, and a sensitivity of Low (about 270±10V) (power source frequency (50/60Hz)), using a Peristaltic sipper, SHP-820. It is also possible to perform the measurement using another fluorescent spectrophotometer, but in such a case, it is necessary to adjust the measurement conditions such as sensitivity to the conditions specified in the subject application.

In the water-containing fluid composition according to this embodiment, the processed lysozyme and/or the salt thereof is stored in the water-containing fluid composition while being in contact with the polar organic solvent. That is, in the water-containing fluid composition according to this embodiment, the processed lysozyme and/or the salt thereof is dissolved in the water-containing fluid composition and therefore can exist in the water-containing fluid composition while being in contact with the polar organic solvent.

The inventors have arrived at surprising understandings that a specific processed lysozyme and/or a salt thereof has a norovirus inactivating action, and a reduction in the norovirus inactivating action can be suppressed when the processed lysozyme and/or the salt thereof in the water-containing fluid composition according to this embodiment is dissolved in the water-containing fluid composition while being in contact with a polar organic solvent (particularly, a water-soluble alcohol such as ethanol), thereby creating the water-containing fluid composition according to this embodiment.

### [Processed lysozyme and/or salt thereof]

In the present invention, the "lysozyme" means a protein having a property of hydrolyzing β-1,4 bonds between N-acetylglucosamine and N-acetylmuramic acid. Further, in the present invention, the "processed lysozyme" is a lysozyme modified product having a three-dimensional structure that is different from that of the lysozyme and/or the salt thereof.

Lysozymes are widely present in the living world such as eggs, tissues and body fluids of animals, and plants, and are roughly classified into the following 5 types of families depending on their substrate specificity and structure.
1. Lysozymes (Bacteria type)
2. Lysozymes (Chicken type)
3. Lysozymes (Goose type)
4. Lysozymes (Phage type; V type)
5. Lysozymes (CH type)

In the water-containing fluid composition according to this embodiment, any of the aforementioned 5 types of families can be used as a raw material for the processed lysozyme and/or the salt thereof. Among these lysozymes, Lysozymes (Chicken type) such as egg white lysozymes and human lysozymes are preferable as a lysozyme that is a raw material. In the water-containing fluid composition according to this embodiment, it is more preferable that the processed lysozyme and/or the salt thereof contain a lysozyme derived from an egg white lysozyme as a raw material, for more excellent norovirus inactivating action, low cost, and ease of availability.

Further, examples of the salt of the processed lysozyme include salts that are acceptable as food additives or are pharmaceutically acceptable, and the examples include salts of inorganic acids such as hydrochloric acid, carbonic acid, phosphoric acid, boric acid, hexametaphosphoric acid, nitric acid, and sulfuric acid, and salts of organic acids such as citric acid, tartaric acid, succinic acid, malic acid, acetic acid, glutamic acid, glycerophosphoric acid, and gluconic acid. Among the salts of lysozyme, the salts of inorganic acids are preferable, and hydrochloride is more preferable in that it is widely used as antiphlogistics, and its safety is established.

The water-containing fluid composition according to this embodiment can contain at least one type selected from the processed lysozyme and/or the salt thereof. Further, in the case where a plurality of types of the processed lysozyme and/or the salt thereof are contained in the water-containing fluid composition according to this embodiment, the concentration of the processed lysozyme and/or the salt thereof (in terms of solid content) means the concentration of the total amount of the processed lysozyme and/or the salt thereof (in terms of solid content).

### (Fluorescence intensity)

The fluorescence intensity of the processed lysozyme and/or the salt thereof specified in the present invention is an index of the surface hydrophobicity of the processed lysozyme and/or the salt thereof. That is, the higher the fluorescence intensity of the processed lysozyme and/or the salt thereof specified in the present invention, the higher the surface hydrophobicity of the processed lysozyme and/or the salt thereof, and it can be said that the surface hydrophobicity of the processed lysozyme and/or the salt thereof is high.

For higher surface hydrophobicity and more excellent norovirus inactivating action, the fluorescence intensity of the processed lysozyme and/or the salt thereof, specified by the following definition, is preferably 4000 or more, more preferably 5000 or more, and is generally 10000 or less.

The fluorescence intensity is a value of the fluorescence intensity measured, under conditions of an excitation wavelength of 390 nm (excitation bandwidth of 10 nm) and a fluorescence wavelength of 470 nm (fluorescence bandwidth of 10 nm), for a solution obtained by adding 25 µL of an 8 mM solution of 1,8-anilinonaphthalenesulfonic acid in methanol to 5 mL of a diluted solution obtained by diluting with a phosphate buffer solution (pH 7.0) to a concentration in terms of solid content of the processed lysozyme and/or the salt thereof of 0.05 mass% and a concentration of the phosphate of 0.2 M, and thereafter allowing the solution to react at room temperature for 30 minutes. Specifically, for example, in the case of measuring the fluorescence intensity of an aqueous solution containing 1 mass% in terms of solid content of the processed lysozyme and/or the salt thereof, the dilution with a phosphate buffer solution to a concentration in terms of solid content of the processed lysozyme and/or the salt thereof of 0.05 mass% and a concentration of the phosphate of 0.2 M means adjustment by diluting 5g of the aqueous solution and 80 mL of a 0.25 M phosphate buffer solution, after being put into a 100 mL volumetric flask, with purified water to 100 mL.

### (Concentration)

In the water-containing fluid composition according to this embodiment, the concentration in terms of solid content of the processed lysozyme and/or the salt thereof is preferably 0.1 mass% or more and 10 mass% or less, more preferably 0.2 mass% or more, further preferably more than 0.5 mass%, for example, 0.6 mass% or more, most preferably 0.7 mass% or more, and is preferably 5 mass% or less, more preferably 4 mass% or less, further preferably 2 mass% or less, in that the reduction in the norovirus inactivating action can be suppressed more reliably.

### (Anti-norovirus activity of processed lysozyme and/or salt thereof)

The processed lysozyme and/or the salt thereof according to this embodiment has a norovirus inactivating action. More specifically, it is preferable that the processed lysozyme and/or the salt thereof according to this embodiment have an anti-norovirus activity specified below of 2.0 or more.

The anti-norovirus activity of the processed lysozyme and/or the salt thereof: a value obtained by subtracting the logarithm of infectivity of a norovirus, mixed solution obtained by mixing a norovirus solution with a 2 mass% aqueous solution of the processed lysozyme and/or the salt thereof in equal amounts after the norovirus mixed solution is allowed to stand at room temperature for one minute, from the logarithm of infectivity of the norovirus mixed solution before being allowed to stand.

### (Dimer and trimer)

The processed lysozyme or salt thereof can contain an approximately 29-KDa protein (KDa = 10³Da) and/or an approximately 36.5-KDa protein. That is, the water-containing fluid composition according to this embodiment can contain an approximately 29-KDa protein and/or an approximately 36.5-KDa protein. The fact that an approximately 29-KDa protein and/or an approximately 36.5-KDa protein is contained in the water-containing fluid composition according to this embodiment and the processed lysozyme or the salt thereof can be confirmed by electrophoresis shown in the later-described examples. The electrophoresis can be performed using a commercially available electrophoresis kit.

The approximately 29-KDa protein is inferred to be a dimer of the processed lysozyme, and the approximately 36.5-KDa protein is inferred to be a trimer of the processed lysozyme.

It is inferred that the processed lysozyme and/or the salt thereof is allowed to exist in the water-containing fluid composition while having high surface hydrophobicity by the approximately 29-KDa protein and/or the approximately 36.5-KDa protein being contained in the water-containing fluid composition according to this embodiment.

### (Production method)

The processed lysozyme and/or the salt thereof can be produced by performing the first heating step, the second heating step, and the third heating step described in "Method for producing processed lysozyme and/or salt thereof" according to the later-described embodiment.

### [Polar organic solvent]

The water-containing fluid composition according to this embodiment contains a polar organic solvent. In the present invention, the "polar organic solvent" means an organic solvent having affinity to water, more specifically, an organic solvent miscible with water. Examples of the polar organic solvent include monovalent alcohols having 1 or more and 4 or less carbon atoms (preferably 3 or less) such as ethanol, methanol, 1-propanol, 2-propanol, and n-butanol, water-soluble alcohols (alcohol-based solvents) including polyhydric alcohols such as glycerin, ethylene glycol, diethylene glycol, propylene glycol, polyethylene glycol, butylene glycol, dipropylene glycol, and sorbitol, ketone-based solvents such as acetone and methyl ethyl ketone, dioxane, tetrahydrofuran, acetonitrile, and N-methylpyrrolidone, and one of these can be used alone, or two or more of them can be used in combination. Among these, for low burden to living bodies, it is preferable for the polar organic solvent to contain an alcohol-based solvent, and it is more preferable to contain ethanol.

In the water-containing fluid composition according to this embodiment, the concentration of the polar organic solvent in the water-containing fluid composition according to this embodiment is 55 mass% or more and 90 mass% or less, preferably 60 mass% or more, more preferably 65 mass% or more, in that the solubility of the processed lysozymes and/or the salt thereof is excellent, and the reduction in the norovirus inactivating action can be suppressed more reliably. Meanwhile, the concentration is preferably 85 mass% or less, more preferably 80 mass% or less.

In the case where the water-containing fluid composition according to this embodiment contains ethanol as the polar organic solvent, the concentration of ethanol in the water-containing fluid composition according to this embodiment can be 55 mass% or more and 90 mass% or less, preferably 60 mass% or more, more preferably 65 mass% or more, and is preferably 85 mass% or less, more preferably 80 mass% or less, in that the reduction in the norovirus inactivating action can be suppressed more reliably.

### [Norovirus inactivating action]

In the present invention, the norovirus inactivating action can be evaluated by a system using the method described in the later-described examples (that is, mouse cells infected with norovirus).

More specifically, the water-containing fluid composition according to this embodiment has a norovirus inactivating action. More specifically, the water-containing fluid composition according to this embodiment can have an anti-norovirus activity specified below of 3.0 or more. It is preferable that the anti-norovirus activity of the water-containing fluid composition in the present invention be a value measured for a water-containing fluid composition in which the concentration (in terms of solid content) of the processed lysozyme is 0.1 mass% or more and 10 mass% or less. In this case, the water.-containing fluid composition according to this embodiment can contain the processed lysozyme or the salt thereof containing an approximately 29-KDa protein and/or an approximately "36.5-KDa protein.

The anti-norovirus activity of the water-containing fluid composition: a value obtained by subtracting the logarithm of infectivity of a norovirus mixed solution obtained by mixing 1 part by mass of the water-containing fluid composition with respect to 0.1 parts by mass of a norovirus solution after being allowed to stand at room temperature for one minute, from the logarithm of infectivity of the norovirus mixed solution before being allowed to stand.

### [Maintaining norovirus, inactivating action]

Further, the water-containing fluid composition according to this embodiment can maintain the norovirus inactivating action after long-term storage. More specifically, the water-containing fluid composition according to this embodiment can have an anti-norovirus activity after storage at 40°Cx75%RH for 4 weeks of 50% or more (preferably 70% or more and generally 100% or less) of the anti-norovirus activity before the storage.

### [Water-soluble alcohol other than ethanol]

The water-containing fluid composition according to this embodiment can further contain a water-soluble alcohol other than ethanol. Examples of the water-soluble alcohol other than ethanol are as described above. The water-soluble alcohol other than ethanol can function as the polar organic solvent. In the case of using the water-soluble alcohol other than ethanol, the water-soluble alcohol other than ethanol can be used alone, or two or more types thereof can be used in combination.

Since the processed lysozyme and/or the salt thereof has excellent water solubility and excellent solubility in a polar organic solvent (particularly ethanol), when the water-containing fluid composition according to this embodiment further contains at least one selected from water-soluble alcohols other than ethanol, the solubility of the processed lysozyme and/or the salt thereof in the polar organic solvent (particularly ethanol) can be further enhanced, as a result of which the contact between the polar organic solvent and the processed lysozyme and/or the salt thereof in the water-containing fluid composition can be secured.

Among these, for low burden to living bodies, it is preferable that the water-containing fluid composition according to this embodiment contain water and a water-soluble alcohol other than ethanol or any one of them in addition to ethanol.

In the case where the water-containing fluid composition according to this embodiment contains a water-soluble alcohol other than ethanol, the content thereof is generally 0.1 mass% or more and 20 mass% or less, preferably 0.5 mass% or more, and preferably 10 mass% or less. In the case where the water-containing fluid composition according to this embodiment contains ethanol and a water-soluble alcohol other than ethanol, it is preferable that the content of the water-soluble alcohol with respect to the content of the ethanol is 1/700 or more and 1/5 or less, in that the solubility of the processed lysozyme and/or the salt thereof in the water-containing fluid composition can be further enhanced.

In the case where the water-containing fluid composition according to this embodiment contains water, the water content in the water-containing fluid composition according to this embodiment is generally 10 mass% or more and 50 mass% or less, preferably 15 mass% or more, more preferably 20 mass% or more, and preferably 40 mass% or less, more preferably 30 mass% or less. In the case where the water-containing fluid composition according to this embodiment contains ethanol, it is preferable that the water content with respect to the content of ethanol is preferably 1/3 or more and 1/1 or less, in that the solubility of the processed lysozyme and/or the salt thereof can be further enhanced.

### [Other materials]

The water-containing fluid composition according to this embodiment can contain a compound material other than the processed lysozyme, the polar organic solvent, and water.

Examples of the other material include bacteriostatic agents such as glycine, organic acids, sodium benzoate, sodium sorbate, sodium propionate, sodium dehydroate, paraoxybenzoic acid ester, sodium sulfite, EDTA, benzalkonium chloride, benzethonium chloride, chlorhexidine gluconate, alkyldiaminoethylglycine chloride, iodine tincture, povidone-iodine, cetyl benzalkonium oxide, triclosan, chloroxylenol, isopropylmethylphenol, ε-polylysine, lactoferrin, nisin, bacteriocin, Udo (Aralia cordata) extract, Egonoki (Styrax japonica) extract, Kawaxayomogi (Artemisia capillaris) extract, enzymatically degraded Hatomugi (Coix lacryma-jobi) extract, Shirako (milt) protein extract, thujaplicin, and a decomposed product of pectin, which can be used appropriately in combination.

The water-containing fluid composition according to this embodiment may further contain additives including a pH adjuster such as citric acid, sodium citrate, sodium hydroxide, and triethanolamine, an antioxidant such as tocopherol acetate ester, and a thickener such as carboxyvinyl polymer and hydroxyethyl cellulose, and a surfactant such as glycerin fatty acid ester and sucrose fatty acid ester, as needed.

### <Dosage form>

The viscosity of the water-containing fluid composition according to this embodiment can be appropriately set corresponding to the use method. For example, a liquid formulation having a sprayable viscosity is employed, and the liquid formulation is packed into a container such as a trigger sprayer, a pump container, a squeeze container, and an aerosol container, so that droplets containing the water-containing fluid composition according to this embodiment are sprayed to hand fingers, foods, kitchenware, residential environment, vomits, excrements, and the like, thereby enabling inactivation of the virus to be performed conveniently. That is, the aforementioned container contains the water-containing fluid composition according to this embodiment.

Further, immersion of the inactivation target (such as hand fingers, foods, medical equipment, medical appliance, kitchenware, and residential environment) into the water-containing fluid composition according to this embodiment enables inactivation of the virus to be performed conveniently.

Further, the water-containing fluid composition according to this embodiment can be used in the form of a sheet obtained by impregnating a non-woven fabric with the water-containing fluid composition. That is, the aforementioned non-woven fabric is impregnated with the water-containing fluid composition according to this embodiment. In this case, examples of materials for the non-woven fabric include paper and fibers. By bringing the sheet obtained by impregnating the non-woven fabric with the water-containing fluid composition according to this embodiment into contact with the surface of the target, inactivation of the virus can be performed conveniently.

Further, when the water-containing fluid composition according to this embodiment is prepared as a lotion, it can be used as cosmetics, or skin external preparations that are pharmaceuticals or quasi drugs.

### [One example]

The water-containing fluid composition according to this embodiment is, for example, a water-containing fluid composition containing a processed lysozyme and/or a salt thereof, wherein the content of the polar organic solvent can be 55 mass% or more and 90 mass% or less, and the concentration of the processed lysozyme and/or the salt thereof can be 0.1 mass% or more and 10 mass% or less. In this case, the processed lysozyme and/or the salt thereof is dissolved in the water-containing fluid composition while being in contact with the polar organic solvent.

In this case, the polar organic solvent contains ethanol and can be 55 mass% or more and 90 mass% or less of the ethanol.

Further, in this case, the water-containing fluid composition can contain an approximately 29-KDa protein and/or an approximately 36.5-KDa protein.

Further, in this case, the water containing fluid composition can have an anti-norovirus activity specified below of 3.0 or more. The anti-norovirus activity of the water--containing fluid composition: a value obtained by subtracting the logarithm of infectivity of a norovirus mixed solution obtained by mixing 1 part by mass of the water-containing fluid composition with respect to 0.1 parts by mass of a norovirus solution after being allowed to stand at room temperature for one minute, from the logarithm of infectivity of the norovirus, mixed solution before being allowed to stand.

Further, the water-containing fluid composition can have an anti-norovirus activity after storage at 40°Cx75%RH for 4 weeks of 50% or more (preferably 70% or more and is generally 100% or less) of the anti-norovirus activity before the storage.

### <Method for producing processed lysozyme and/or salt thereof>

The method for producing the processed lysozyme and/or the salt thereof according to one embodiment of the present invention, the processed lysozyme and/or the salt thereof having a fluorescence intensity, as specified by the aforementioned definition, of 4000 or more, comprises: a first heating step of heating a lysozyme aqueous solution having a light transmittance at 660-nm wavelength of more than 70%, a pH of 5.0 or more and 7.0 or less, and a concentration in terms of solid content of the lysozyme and/or the salt thereof of 0.5 mass% or more and 7 mass% or less, until the light transmittance at 660-nm wavelength of the aqueous solution reaches 70% (which will be hereinafter simply referred to also as "first heating step"); a second heating step of heating the aqueous solution after the first heating step until the light transmittance at 660-nm wavelength of the aqueous solution reaches a minimum value of less than 70% and thereafter heating the aqueous solution up to 70% (which will be hereinafter simply referred to also as "second heating step"); and a third heating step of further heating the aqueous solution after the second heating step in the state where the light transmittance at 660-nm wavelength of the aqueous solution is more than 70% (which will be hereinafter simply referred to also as "third heating step").

According to the method for producing the processed lysozyme and/or the salt thereof according to this embodiment, a processed lysozyme and/or a salt thereof having a fluorescence intensity, as specified by the aforementioned definition, of 4000 or more can be obtained by the first heating step, the second heating step, and the third heating step, and a water-containing fluid composition which contains the processed lysozyme and/or the salt thereof and has a norovirus inactivating action and in which a reduction in the norovirus inactivating action is suppressed can be obtained by mixing the processed lysozyme and/or the salt thereof obtained by the aforementioned method for producing the lysozyme with a polar organic solvent.

### [Heating mechanism]

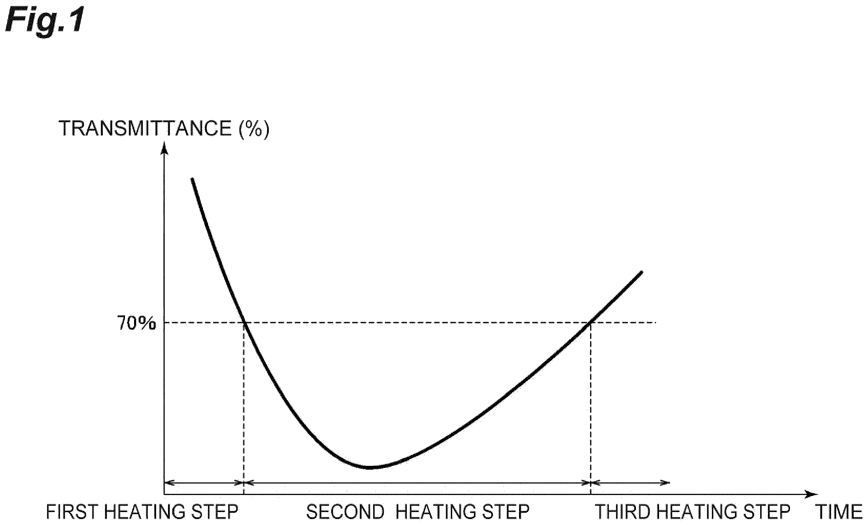

Figure 1 is a graph schematically showing the relationship between the heating time in the first heating step, the second heating step, and the third heating step, and the transmittance in the method for producing the processed lysozyme and/or the salt thereof according to this embodiment. The inventors have found that, when heating the aqueous solution containing the lysozyme, the light transmittance at 660-nm wavelength changes according to the heating time as shown in Figure 1. It is inferred that such a change in transmittance is mainly caused by a change in surface hydrophobicity and a change in water solubility (decrease in water solubility and subsequent increase in water solubility) of the processed lysozyme to be obtained.

As shown in Figure 1, the aqueous solution has a light transmittance at 660-nm wavelength of more than 70%, which is then decreased to 70% by the first heating step. More specifically, the transparency of the aqueous solution is decreased by the first heating step.

Further, after the light transmittance at 660-nm wavelength of the aqueous solution, which has been decreased to 70% by the first heating step, is further decreased by the second heating step, it can be increased again to 70%. More specifically, as shown in Figure 1, the light transmittance at 660-nm wavelength of the aqueous solution is decreased by the second heating step, and after having decreased to the minimum value of less than 70%, it is increased up to 70%. That is, in this case, after white turbidity and/or precipitation in the aqueous solution is observed by visual inspection (in other words, after the transmittance (transparency) of the aqueous solution is decreased), the white turbidity and/or precipitation can gradually disappear (in other words, the transmittance (transparency) of the aqueous solution can be increased).

Further, in the state where the light transmittance at 660-nm wavelength of the aqueous solution, which has reached 70% by the second heating step, is more than 70%, the aqueous solution is further heated by the third heating step. More specifically, it is preferable that white turbidity and/or precipitation do not occur in the aqueous solution in the third heating step (in other words, the transmittance (transparency) of the aqueous solution is maintained).

### [First heating step]

The heating target in the first heating step is an aqueous solution of the lysozyme and/or the salt thereof having a light transmittance at 660-nm wavelength of more than 70% (preferably 80% or more, more preferably 90% or more, and is generally 100% or less), a pH of 5.0 or more and 7.0 or less, and a concentration in terms of solid content of the lysozyme and/or the salt thereof of 0.5 mass% or more and 7 mass% or less.

The fact that the light transmittance at 660-nm wavelength of the aqueous solution having a light transmittance of more than 70% becomes 70% by the first heating step means that the transmittance of the aqueous solution decreases, in which white turbidity can be observed in the aqueous solution by visual inspection, for example.

That is, it is inferred that, in the first heating step, the surface hydrophobicity of the three-dimensional structure and/or the surface of the lysozyme in the aqueous solution increases, the hydrophobic portions pull each other and aggregate together, and the solubility of the lysozyme in the aqueous solution decreases, as a result of which the light transmittance at 660-nm wavelength of the aqueous solution of more than 70% decreases to 70%.

### (Raw material)

As the lysozyme and/or the salt thereof that is a raw material used in the aqueous solution in the first heating step, the lysozyme and/or the salt thereof as exemplified in "Water-containing fluid composition" described above can be used. For low cost and ease of availability, it is preferable that the lysozyme and/or the salt thereof that is a raw material be an egg white lysozyme.

### (Concentration of raw material)

The concentration of the lysozyme in the aqueous solution in the first heating step is preferably 0.5 mass% or more, more preferably 1 mass% or more, and is preferably 7 mass% or less, more preferably 5 mass% or less, in that the three-dimensional structure of the lysozyme can be reliably changed, and the norovirus inactivating action can be increased by preventing the aggregation in the lysozyme.

### (Aqueous solution of lysozyme and/or salt thereof)

The solvent constituting the aqueous solution of the lysozyme and/or the salt thereof is water, but an organic solvent that is miscible with water may be used without affecting the solubility of the lysozyme and/or salt thereof in water. The ratio of water in the solvent constituting the aqueous solution of the lysozyme and/or the salt thereof is generally 80 mass% or more and 100 mass% or less. Further, in the case where the solvent constituting the aqueous solution of the lysozyme and/or the salt thereof contains an organic solvent that is miscible with water, the ratio of the organic solvent in the solvent constituting the aqueous solution of the lysozyme and/or the salt thereof is generally 1 mass% or more 20 mass% or less.

The organic solvent needs only to be miscible with water, and examples thereof include alcohol-based solvents such as methanol, ethanol, 1-propanol, 2-propanol, ethylene glycol, propylene glycol, and glycerin, ketone-based solvents such as acetone and methyl ethyl ketone, acetonitrile, tetrahydrofuran, and 1,4-dioxane, and one of these can be used alone, or two or more of these can be used in combination.

### (pH)

The pH of the aqueous solution in the first heating step, the second heating step, and the third heating step is preferably 5.0 or more, more preferably more than 5.0, further preferably 5.5 or more, and is preferably 7.0 or less, more preferably 6.5 or less, in that the aggregation in the lysozyme can be prevented, and the surface hydrophobicity of the processed lysozyme and/or the salt thereof to be obtained can be enhanced.

Further, the pH of the aqueous solution may be adjusted to the aforementioned range by using an acid (for example, an inorganic acid such as hydrochloric acid, sulfuric acid, and nitric acid, and an organic acid such as citric acid, acetic acid, and phosphoric acid), an alkali (for example, an inorganic base such as sodium hydroxide and potassium hydroxide), or a buffer solution (for example, acetic acid buffer solution), as needed.

For example, in the case where the lysozyme is a salt of lysozyme (such as lysozyme chloride), it is preferable that the first heating step be performed, after the pH of the aqueous solution is adjusted to the aforementioned range using an acid, an alkali, or a buffer solution.

### [Second heating step]

In the second heating step, the fact that the light transmittance at 660-nm wavelength of the aqueous solution obtained in the first heating step changes from 70% to the minimum value of less than 70% and is further heated to 70% means that the transmittance of the aqueous solution obtained in the first heating step, after being further decreased, turns to increase, and it can be confirmed by visual inspection that the aqueous solution is gradually becoming transparent.

That is, it is inferred that, as the three-dimensional structure of the lysozyme in the aqueous solution further changes, and the surface hydrophobicity of lysozymes further increase in the second heating step, the water solubility of the lysozyme, after having decreased once, increases, as a result of which the light transmittance at 660-nm wavelength of the aqueous solution becomes 70%.

The mechanism by which the water solubility of the lysozyme, after having decreased once, increases as the surface hydrophobicity of lysozymes further increases in the second heating step is not clear, but it is inferred that solubility of the lysozyme in the aqueous solution increases because the aggregation in the lysozyme that has occurred in the first heating step further proceeds by the second heating step, aggregates in the lysozyme are subsequently bound to each other to transform into a linear aggregate, and the aggregate is dissolved in the aqueous solution.

As shown in Figure 1, the light transmittance at 660-nm wavelength of the aqueous solution, after having decreased from 70% to the minimum value of less than 70%, increases up to 70% by the second heating step. The minimum value of the light transmittance at 660-nm wavelength of the aqueous solution in the second heating step is preferably less than 60%, more preferably less than 50% (generally 0% or more), in that the three-dimensional structure of the lysozyme in the aqueous solution can be changed more reliably.

The second heating step can be performed subsequently to the first heating step. That is, the second heating step can be performed continuously with the first heating step.

### [Third heating step]

In the third heating step, the aqueous solution is further heated after the second heating step, in the state where the light transmittance at 660-nm wavelength of the aqueous solution is more than 70%. The processed lysozyme can be obtained by the first heating step, the second heating step, and the third heating step.

That is, it is inferred that the three-dimensional structure of the lysozyme in the aqueous solution further changes in the third heating step, and the surface hydrophobicity of lysozymes is further enhanced while the water solubility of the lysozyme is maintained, as a result of which the value of the light transmittance at 660-nm wavelength of the aqueous solution of more than 70% can be maintained.

The heating in the third heating step is more preferably performed until the light transmittance at 660-nm wavelength of the aqueous solution reaches 75% or more, more preferably 80% or more (generally 100% or less), in that the three-dimensional structure of the lysozyme in the aqueous solution is changed more reliably.

Further, the heating in the third heating step is preferably performed until the light transmittance at 660-nm wavelength of the aqueous solution obtained in the third heating step after being filtered through a 0.45 µm membrane filter and mixed with ethanol at a mass ratio of 1:1 reaches 85% or more, more preferably 90% or more (generally 100% or less).

The fact that the light transmittance at 660-nm wavelength of the aqueous solution obtained in the third heating step after being filtered through a 0.45 µm membrane filter and mixed with ethanol at a mass ratio of 1:1 reaches 85% or more can be an index that indicates the processed lysozyme and/or the salt thereof having a fluorescence intensity, as specified by the aforementioned definition, of 4000 or more has been obtained.

The third heating step can be performed subsequently to the second heating step. That is, the third heating step can be performed continuously with the first heating step and the second heating step.

The aqueous solution that has been heated to be transparent while the transmittance is more than 70% in the third heating step can maintain the transmittance (in other words, can maintain the transparency) after the temperature is returned to room temperature (for example, 25°C). It is inferred that this is because the three-dimensional structure of the linear aggregate in the form of the lysozyme or the like contained in the aqueous solution is maintained also at room temperature.

### (Heating temperature and heating time)

The heating is performed so that the product center temperature of the aqueous solution in the first heating step, the second heating step, and the third heating step is preferably 70°C or more, in that a high yield can be achieved in the production of the processed lysozyme and/or the salt thereof in the method for producing the water-containing fluid composition according to this embodiment, and the aqueous solution can be adjusted to have a transmittance as specified in each of the first heating step, the second heating step, and the third heating step, and the product center temperature is more preferably 80°C or more, further preferably 90°C or more, and may be generally 130°C, further may be about 100°C or less, in that the production time can be reduced due to short heating time. The heating time in the first heating step, the second heating step, and the third heating step can be appropriately determined in such a way as to satisfy the transmittance specified in each step corresponding to the heating temperature and the processing amount.

More specifically, it is preferable that, in the case of performing the first heating step and the second heating step continuously at the same heating temperature, it is preferable that the total of the heating time in the first heating step and the heating time in the second heating step be 5 minutes or more and 120 minutes or less when the product center temperature is 70°C or more and 75°C or less, the total time be 5 minutes or more and 75 minutes or less when the product center temperature is more than 75°C and 80°C or less, the total time be 5 minutes or more and 65 minutes or less when the product center temperature is more than 80°C and 85°C or less, the total time be 5 minutes or more and 45 minutes or less when the product center temperature is more than 85°C and 90°C or less, the total time be 5 minutes or more 35 minutes or less when the product center temperature is more than 90°C and 95°C or less, and the total time be 5 minutes or more 20 minutes or less when the product center temperature is more than 95°C and 100°C or less.

It is preferable that the heating time in the third heating step be 5 minutes or more and 600 minutes or less when the product center temperature is 70°C or more and 75°C or less, the heating time be 5 minutes or more and 360 minutes or less when the product center temperature is more than 75°C and 80°C or less, the heating time be 5 minutes or more and 240 minutes or less when the product center temperature is more than 80°C and 85°C or less, the heating time be 5 minutes or more and 195 minutes or less when the product center temperature is more than 85°C and 90°C or less, the heating time be 5 minutes or more and 150 minutes or less when the product center temperature is more than 90°C and 95 °C or less, and the heating time be 5 minutes or more and 100 minutes or less when the product center temperature is more than 95 °C and 100°C or less.

It is preferable that, in the case of performing the first heating step, the second heating step, and the third heating step continuously at the same heating temperature, the total of the heating time in the first heating step, the heating time in the second heating step, and the heating time in the third heating step be 125 minutes or more and 720 minutes or less when the product center temperature is 70°C or more and 75°C or less, the total time be 80 minutes or more and 435 minutes or less when the product center temperature is more than 75°C and 80°C or less, the total time be 70 minutes or more and 305 minutes or less when the product center temperature is more than 80°C and 85°C or less, the total time be 50 minutes or more and 240 minutes or less when the product center temperature is more than 85°C and 90°C or less, the total time be 40 minutes or more and 185 minutes or less when the product center temperature is more than 90°C and 95°C or less, and the total time be 25 minutes or more and 120 minutes or less when the product center temperature is more than 95°C and 100°C or less.

### (Spray drying/freeze drying)

The method for producing the processed lysozyme and/or the salt thereof according to this embodiment can further comprise a step of obtaining the processed lysozyme and/or the salt thereof in powder form by spray drying or freeze drying the aqueous solution after the third heating step.

The spray drying and freeze drying may be performed according to a conventional method.

### <Method for producing water-containing fluid composition>

A method for producing a water-containing fluid composition according to one embodiment of the present invention comprises a step of obtaining a water-containing fluid composition having a concentration of a polar organic solvent of 55 mass% or more and 90 mass% or less by mixing the processed lysozyme and/or the salt thereof (more specifically, the processed lysozyme and/or the salt thereof obtained by the aforementioned method for producing the processed lysozyme and/or the salt thereof) with the polar organic solvent. That is, the water-containing fluid composition according to the aforementioned embodiment can be obtained by the aforementioned production method.

In the production method according to this embodiment, it is preferable to mix the polar organic solvent with the processed lysozyme and/or the salt thereof in the step of obtaining the water-containing fluid composition, so that the concentration in terms of solid content of the processed lysozyme and/or the salt thereof in the water-containing fluid composition is preferably 0.1 mass% or more and 10 mass% or less (more preferably 0.2 mass% or more, further preferably more than 0.5 mass%, for example, 0.6 mass% or more, further preferably 0.7 mass% or more, and more preferably 4 mass% or less, further preferably 2 mass% or less), in that the reduction in the norovirus inactivating action can be suppressed more reliably. In this case, other components as exemplified in "Water-containing fluid composition" according to the aforementioned embodiment can be further mixed.

Further, in the production method according to this embodiment, it is preferable, for example, that the polar organic solvent contain ethanol, and the polar organic solvent be mixed with the processed lysozyme and/or the salt thereof in the step of obtaining the water-containing fluid composition, so that the concentration of ethanol in the water-containing fluid composition is preferably 55 mass% or more and 90 mass% or less (more preferably 60 mass% or more, further preferably 65 mass% or more, and more preferably 85 mass% or less, further preferably 80 mass% or less), in that the reduction in the norovirus inactivating effect can be suppressed more reliably.

### <Method for using water-containing fluid composition (virus inactivating method)>

Methods (methods for inactivating viruses) such as spraying or applying the water-containing fluid composition according to this embodiment to the target region in which the viruses (norovirus) are inactivated, mixing the water-containing fluid composition with the target in which the viruses are inactivated, and ingesting the water-containing fluid composition can be employed.

### <Action and effect>

The water-containing fluid composition according to this embodiment contains the processed lysozyme and/or the salt thereof and therefore has a virus inactivating action. In particular, the water-containing fluid composition according to the embodiment has a norovirus inactivating action by containing the processed lysozyme and/or the salt thereof, and the processed lysozyme and/or the salt thereof is dissolved in the water-containing fluid composition while being in contact with the polar organic solvent, so that the reduction in the norovirus inactivating action can be suppressed, and therefore the norovirus inactivating action is maintained over a long period of time.

### Examples

### [Example 1]

20 g of a lysozyme (egg white lysozyme obtained from chicken egg white as a raw material) was added to 980 g of pure water and was mixed therewith, to prepare a 2 mass% aqueous solution of the lysozyme. Thereafter, the aqueous solution was heated at a product center temperature of 80°C for 10 minutes, as a result of which the light transmittance at 660-nm wavelength of the aqueous solution decreased to 70% (first heating step). Subsequently, after the heating was performed until the light transmittance at 660-nm wavelength of the aqueous solution reaches the minimum value of less than 70% (20%), the aqueous solution was further heated at 80°C for 60 minutes (second heating step). Subsequently, in the state where the light transmittance at 660-nm wavelength of the aqueous solution has increased to exceed 70%, the aqueous solution was heated at a product center temperature of 80°C for 110 minutes (third heating step). Thus, an aqueous solution containing 2 mass% of the processed lysozyme was obtained.

The transmittance of the aqueous solution after the third heating step was 96%, and the transmittance of the aqueous solution that was filtered through a 0.45 µm membrane filter and mixed with ethanol at a mass ratio of 1:1 was 97%.

After the resultant 2 mass% aqueous solution of the processed lysozyme was filtered through a 0.45 µm membrane filter, 25 g of the resultant filtrate was mixed with a polar organic solvent (60 g of ethanol and 1 g of glycerin), 13 g of water, and 1 g of glycerin fatty acid ester, to obtain a water-containing fluid composition of Test number I .

The conditions shown in Table 1 were changed to prepare the processed lysozymes of Test numbers 2 to 22. Subsequently, a water-containing fluid composition (viscosity: 3 mPa·s) containing each processed lysozyme with the composition of Table 1 was prepared.

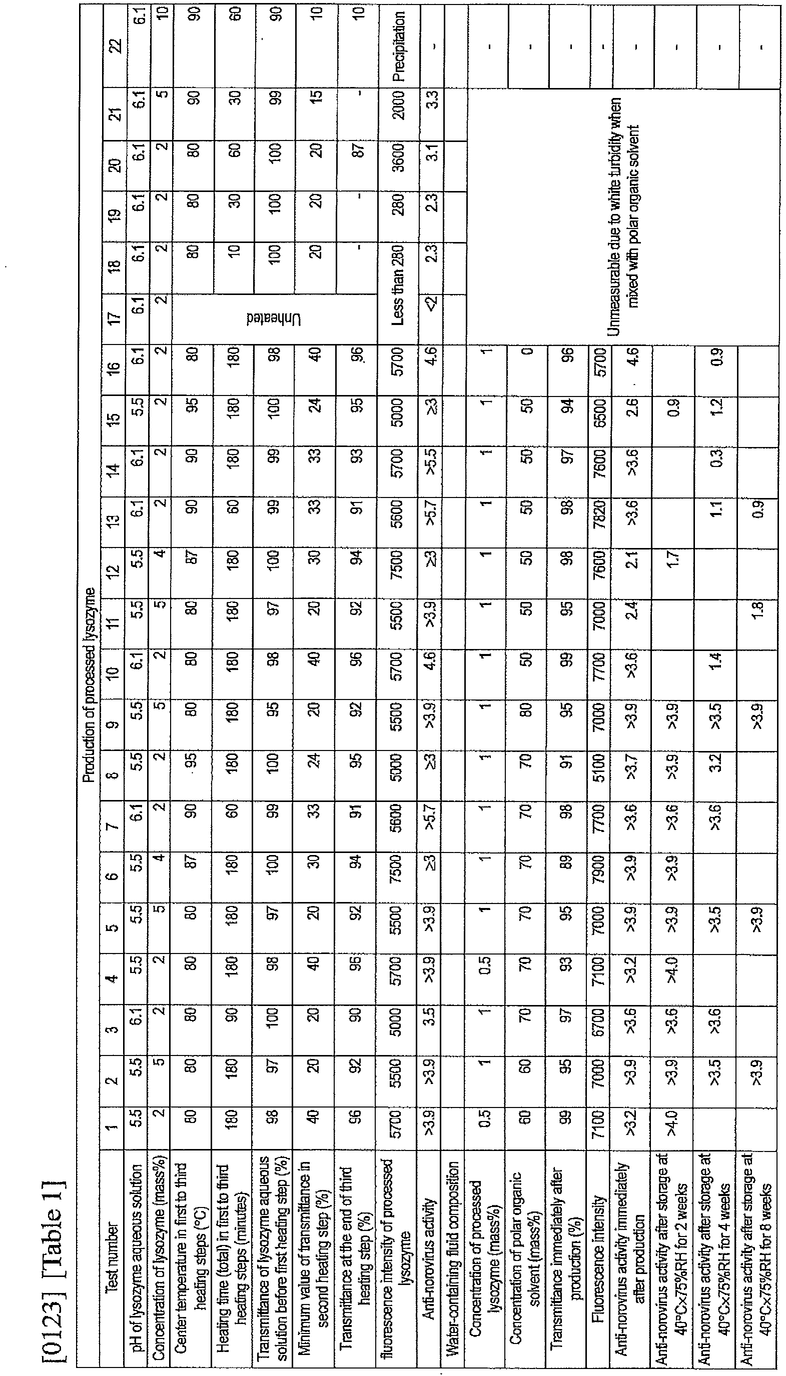

The light transmittance at 660-nm wavelength of the water-containing fluid composition and the aqueous solution in each example of Test numbers 1 to 22 was measured using an absorptiometer ("UV-2450", manufactured by SHIMADZU CORPORATION). In Table 1, the light transmittance at 660-nm wavelength (%) of the aqueous solution before the first heating step, the minimum value (%) of the light transmittance at 660-nm wavelength in the second heating step, and the light transmittance at 660-nm wavelength (%) of the aqueous solution at the end of heating (that is, the light transmittance at 660-nm, wavelength of the aqueous solution after the third heating step) in each example of Test numbers 1 to 22 are shown. In Test numbers 1 to 22, the first heating step, the second heating step, and the third heating step were continuously performed, and the heating time in Table 1 is the total heating time of the first, second, and third heating steps.

In Test numbers 1 to 16, a processed lysozyme was obtained by heating an aqueous solution of a lysozyme and/or a salt thereof having a light transmittance at 660-nm wavelength of the aqueous solution before the heating of more than 70%, a pH of 5.0 or more and 7.0 or less, and a concentration in terms of solid content of the lysozyme and/or the salt thereof of 0.5 mass% or more and 7 mass% or less, until the light transmittance at 660-nm wavelength of the aqueous solution reaches 70% in the first heating step, subsequently heating the aqueous solution after the aqueous solution becomes white turbid once, until the white turbidity gradually disappears so that the transparency is high (the light transmittance at 660-nm wavelength reaches 70%) in the second heating step, and subsequently heating the aqueous solution, while the transparency of the aqueous solution is maintained (more specifically, in the state where the light transmittance at 660-nm wavelength of the aqueous solution is more than 70% is maintained) in the third heating step.

Further, in each production process for obtaining the processed lysozyme of Test numbers 2 to 16, the light transmittance at 660-nm wavelength of the aqueous solution obtained in the third heating step after being filtered through a 0.45 µm membrane filter and mixed with ethanol at a mass ratio of 1:1 was 85% or more.

In Test numbers 18 and 19, the light transmittance of the aqueous solution after having decreased to less than 70% (20%) was not increased again to 70% or more. Further, in Test number 21, the light transmittance of the aqueous solution after having decreased to less than 70% (15%) was increased to 40% but was not increased to 70% or more.

### [Experimental Example 1]

In this experimental example, the norovirus inactivating action and the time-dependent change in the inactivating action of the water-containing fluid composition of Test numbers 1 to 21 were investigated.

### <Evaluation of anti-norovirus activity>

The anti-norovirus activity of the water-containing fluid composition of Test numbers 1 to 21 was evaluated as follows by plaque assay. The anti-norovirus activity of the water-containing fluid composition of each of Test numbers 1 to 21 is a value measured for the water-containing fluid composition having a concentration (in terms of solid content) of the processed lysozyme shown in Table 1.

### (Macrophage cell culture)

(1) A mouse macrophage cell line (RAW264.7 cells) was cultured in a 6 well plate to 60 to 80% of confluence.

### (Preparation of narovirus solution)

(2) Meanwhile, a norovirus solution having a norovirus infectivity (PFU/mL) of about 10⁶ to 10⁷ PFU/mL was prepared as follows.

First, the mouse macrophage cell line (RAW264.7 cell) was cultured to confluence, and 1 mL of a norovirus stock solution was inoculated into the confluent cells, which were cultured under conditions of 37°C and 5%CO₂ for two days. In this case, Murine norovirus strain 1 (MNV-1) (Effect of Food Residues on Norovirus Survival on Stainless Steel Surfaces) was used as the norovirus stock solution.

The MNV 1 was donated by Dr. Herbert W. Virgin at Washington University.

After culture, it was confirmed by visual inspection that the cells were stripped, and the cells were broken by repeating freeze-thaw 4 times to release viruses in the cells. Thereafter, it was dispensed into a 50 mL centrifuge tube and was centrifuged (8000 g for 20 minutes) to obtain a norovirus solution having an infectivity (PFU/mL) of about 10⁶ to 10⁷ PFU/mL. This norovirus solution was stored at -80°C and was thawed for use. The infectivity of this norovirus solution was measured by the following method.

### (Preparation of norovirus mixed solution)

(3) In order to measure the norovirus infectivity of the water-containing fluid composition, after 0.1 part by mass of the norovirus solution obtained in (2) was added to 1 part by mass of the water-containing fluid composition of Test numbers 1 to 21, the mixture was allowed to stand for one minute at room temperature to obtain a mixed solution (norovirus mixed solution) of each water-containing fluid composition with norovirus.

### (Measurement of infectivity)

Next, the infectivity of the norovirus solution prepared above in (2) and the infectivity of the mixed solution after being allowed to stand for one minute were calculated by the following method.

The norovirus mixed solution (or norovirus solution) was diluted 10^{x}-fold and was used as a diluted sample. Note that x is an integer and is a number that enables counting of the number of plaques by visual inspection in (8) described below. That is, the dilution factor was adjusted so that the number of plaques was about 10 or more and 100 or less in (8). Since the cells are modified due to the influence of the residual alcohol in the norovirus mixed solution (or norovirus solution), the mixed solution was not diluted 10-fold.
(4) All the culture liquid on the plate of (1) was removed, and the diluted sample diluted after being allowed to stand for a predetermined time was inoculated into two wells for each at 500 µL/well.
(5) The mouse macrophage cell line (RAW264.7 cells) was incubated for one hour at room temperature while being shaken in such a way as not to be dried so that the mouse macrophage cell line was infected with the norovirus.
(6) All the 500 µL/well inoculum on the plate was removed, and 1.5% Sea Plaque Agarose-DMEM (37°C) was layered at 2 ml/well, and after hardening, it was cultured for two days under conditions of 37°C and 5% CO₂.
(7) A 0.03% neutral red solution as a staining solution was layered on the plate at 2 mL/well after the culture for two days and was incubated for one hour under conditions of 37°C and 5%CO₂.
(8) All the 0.03% neutral red solution was removed after the incubation of (7), and the number of plaques was counted by visual inspection. From the thus obtained number of plaques and the dilution factor, the infectivity (PFU/mL) of the norovirus mixed solution (or norovirus solution) after being allowed to stand for one minute was calculated. As the infectivity of the norovirus mixed solution before being allowed to stand for one minute, a value obtained by multiplying the mixing ratio (0.1/1.1) of the norovirus solution with the water-containing fluid composition in the norovirus mixed solution by the infectivity of the norovirus solution was used.
(9) The anti-norovirus activity of the water-containing fluid composition of Test numbers 1 to 21 was calculated by the following formula.
Anti-norovirus activity = Log₁₀ (infectivity of norovirus mixed solution before being allowed to stand for one minute) - Log₁₀ (infectivity of norovirus mixed solution after being allowed to stand for one minute)

In the water-containing fluid composition of Test numbers 1 to 9, the infectivity after being allowed to stand for one minute was 10⁴ or less, and the anti-norovirus activity calculated from the aforementioned formula was 3 or more, as shown in Table 1. That is, it was confirmed that the water-containing fluid composition of Test numbers 1 to 9 contains the processed lysozyme and/or the salt thereof and has a norovirus inactivating action immediately after the production by having a fluorescence intensity specified above of 5000 or more.

Further, it was confirmed that the processed lysozyme and/or the salt thereof is dissolved in the water-containing fluid composition while being in contact with a polar organic solvent, and the content of the polar organic solvent in the water-containing fluid composition is 55 mass% or more and 90 mass% or less, so that the water-containing fluid composition of Test numbers 1 to 9 has the norovirus inactivating action even after storage at 40°C×75%RH for 2 weeks, 4 weeks, and 8 weeks, where the reduction in the norovirus inactivating action is suppressed.

More specifically, it was confirmed that, in the water-containing fluid composition of Test numbers 1 to 9, the anti-norovirus activity after storage at 40°C×75%RH for 4 weeks is 50% or more (70% or more) with respect to the anti-norovirus activity before the storage (immediately after the production).

In contrast, it was confirmed that, in the water-containing fluid composition of Test numbers 10 to 15, the content of the polar organic solvent is less than 55 mass% (50 mass%), and therefore the norovirus inactivating action is reduced after storage at 40°C×75%RH for 2 weeks, 4 weeks, and 8 weeks. More specifically, it was confirmed that, in the water-containing fluid composition of Test numbers 10 to 15, the anti-norovirus activity after storage at 40°C×75%RH for 4 weeks is reduced to less than 50% with respect to the anti-norovirus activity before the storage (immediately after the production).

Further, it was confirmed that, in the water-containing fluid composition of Test number 16, the content of the polar organic solvent is less than 20 mass% (0 mass%), and therefore the anti-norovirus activity after storage at 40°C×75%RH for 4 weeks is considerably reduced with respect to the anti-norovirus activity before the storage (immediately after the production).

Further, the fluorescence intensity of the processed lysozyme prepared in Test numbers 17 to 21 was less than 4000, and it was confirmed that the anti-norovirus activity is low. Further, when the processed lysozyme prepared in Test numbers 17 to 21 was mixed with the polar organic solvent, white turbidity occurred, and thus the fluorescence intensity and the anti-norovirus activity of the water-containing fluid composition containing the processed lysozyme could not be measured.

An aqueous solution having a concentration of the processed lysozyme of 10 mass% was prepared in Test number 22, where precipitation occurred in the aqueous solution at room temperature (25°C), and the fluorescence intensity and the anti-norovirus activity could not be measured.

### [Experimental Examples 2]

Electrophoresis was performed on the processed lysozyme obtained in this experimental example. In this experimental example, the heating conditions (heating temperature, heating time, and concentration of egg white lysozyme) in Test number 1 were changed to prepare a processed lysozyme.

More specifically, 950 µL of a sample buffer was added to 50 µL of a 2 mass% aqueous solution of egg white lysozyme collected at a heating time of 0 minute, 15 minutes, 30 minutes, 60 minutes, 90 minutes, and 120 minutes at a heating temperature of 80°C, followed by heating at 100°C for 10 minutes and subsequent cooling with ice, and an electrophoretic gel was charged with 10 µL (10 µg as the processed lysozyme). As a sample buffer, a buffer to which 2-mercapto ethanol was not added (non-reduced) was used. As the electrophoretic gel, SDS-PAGEmini (manufactured by TEFCO, with a gel concentration of 4 to 10% and a gel thickness of 1 mm) was used and was migrated at a constant current of 20 mA. As a staining solution, Coomassie Blue R250 was used.

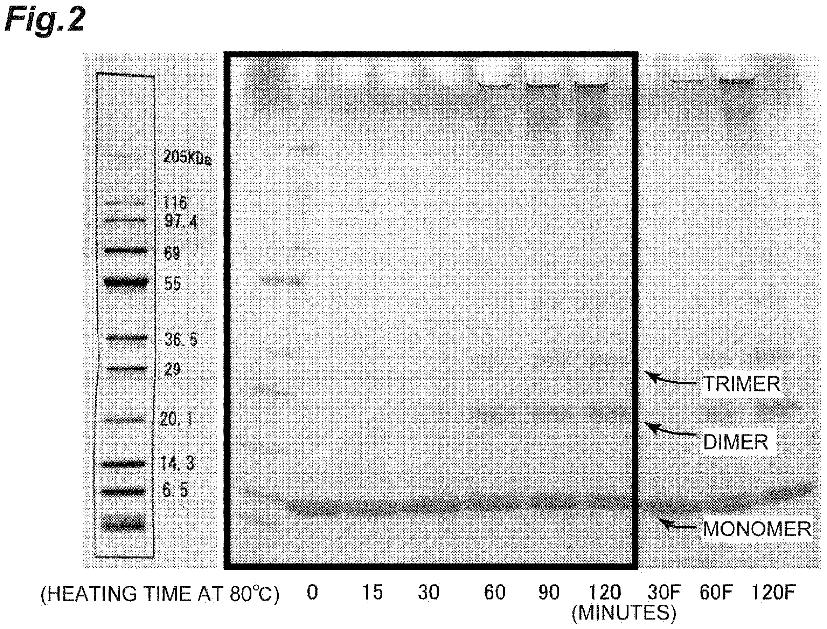

Figure 2 is an image showing the results of the electrophoresis. As shown in Figure 2, at a heating time of 60 minutes, 90 minutes, and 120 minutes, bands showing an approximately 29-KDa protein (inferred to be a dimer) and/or an approximately 36.5-KDa protein (inferred to be a trimer) were clearly detected. Further, it was confirmed that the longer the heating time, the bands showing these proteins are thickened (that is, the amount of the proteins to be produced is increased). Further, it is inferred that, at the same heating temperature, the longer the heating time, the surface hydrophobicity of proteins is increased, so that proteins with higher norovirus inactivating action are obtained.

### [Example 2]

Using the processed lysozyme obtained in Test number 2 of Example 1, the water-containing fluid composition of Test numbers 23 to 26 was obtained in the same manner as in Test number 2, except that the concentration of the processed lysozyme was changed to 0.3 mass%, 0.6 mass%, and 2 mass%. The fluorescence intensity and the anti-norovirus activity of the water-containing fluid composition of Test numbers 23 to 26 were measured by the aforementioned method, as a result of which, the fluorescence intensity was 5000 or more in every case, and the anti-norovirus activity immediately after the production and after storage at 40°C×75%RH for 8 weeks was 3.0 or more in every case.

### [Compounding Example 1]

The water-containing fluid composition obtained in Test number 9 was mixed with the following components at the following compounding ratio to prepare a gel-like disinfectant (water-containing fluid composition).

| | |
|---|---|
| Water-containing fluid composition (Test number 9) | 98 mass% |
| Glycerin | 1 mass% |
| Carboxyvinyl polymer | 1 mass% |

### [Compounding Example 2]

A non-woven fabric was impregnated with a mixture of the water-containing fluid composition obtained in Test number 9 with the following components at the following compounding ratio to prepare wet tissue.

| | |
|---|---|
| Water-containing fluid composition (Test number 5) | 99 mass% |
| PEG-40 hydrogenated castor oil | 1 mass% |

### Industrial Applicability

The water-containing fluid composition of the present invention is useful as disinfectants for disinfecting, for example, hand fingers, bodies, medical appliances, facilities such as schools, hospitals, and welfare facilities, factories, and residences, foods additives, or virus (norovirus) removers.

The explanations of the embodiments according to the present invention are as described above. The present invention includes configurations substantially the same as the configurations described in the embodiments, for example, configurations having the same function, method, and results, or configurations having the same object and results. Further, the present invention includes configurations in which unessential parts of the configurations described in the embodiments are replaced. Further, the present invention includes configurations having the same action and effects or configurations achieving the same object as the configurations described in the embodiments. Further, the present invention includes configurations in which conventional technology is added to the configurations described in the embodiments.

## Claims

1. A water-containing fluid composition comprising a processed lysozyme and/or a salt thereof, the water-containing fluid composition having a fluorescence intensity specified below of 5000 or more,
wherein the processed lysozyme and/or the salt thereof is dissolved in the water-containing fluid composition while being in contact with a polar organic solvent, and
wherein a content of the polar organic solvent in the water-containing fluid composition is 55 mass% or more and 90 mass% or less:
the fluorescence intensity: a fluorescence intensity measured under conditions of an excitation wavelength of 390 nm (excitation bandwidth of 10 nm) and a fluorescence wavelength of 470 nm (fluorescence bandwidth of 10 nm), for a solution obtained by adding 25 µL of an 8 mM solution of 1,8-anilinonaphthalenesulfonic acid in methanol to 5 mL of a diluted solution obtained by diluting the water-containing fluid composition with a phosphate buffer solution (pH 7.0) to a concentration in terms of solid content of the processed lysozyme and/or the salt thereof of 0.05 mass% and a concentration of phosphate of 0.2 M, and thereafter allowing the solution to react at room temperature for 30 minutes.

2. The water-containing fluid composition according to claim 1, wherein an anti-norovirus activity specified below is 3.0 or more,
the anti-norovirus activity: a value obtained by subtracting a logarithm of infectivity of a norovirus mixed solution obtained by mixing 1 part by mass of the water-containing fluid composition with respect to 0.1 parts by mass of a norovirus solution after being allowed to stand at room temperature for one minute, from a logarithm of infectivity of the norovirus mixed solution before being allowed to stand.

3. The water-containing fluid composition according to claim 1 or 2, wherein the fluorescence intensity is 6000 or more.

4. The water-containing fluid composition according to any one of claims 1 to 3, wherein the polar organic solvent comprises ethanol, and wherein a concentration of the ethanol is 55 mass% or more and 90 mass% or less.

5. The water-containing fluid composition according to any one of claims 1 to 4, wherein a concentration in terms of solid content of the processed lysozyme and/or the salt thereof is 0.1 mass% or more and 10 mass% or less.

6. The water-containing fluid composition according to any one of claims 1 to 5, further containing water and a water-soluble alcohol other than ethanol, or either one of them.

7. The water-containing fluid composition according to any one of claims 1 to 6, wherein the processed lysozyme and/or the salt thereof is derived from an egg white lysozyme.

8. The water-containing fluid composition according to any one of claims 1 to 7, being packed into a spray container.

9. The water-containing fluid composition according to any one of claims 1 to 7, being packed into a pump container.

10. The water-containing fluid composition according to any one of claims 1 to 7, wherein a non-woven fabric is impregnated with the water-containing fluid composition.

11. A method for inactivating bacteria and/or viruses, comprising:
a step of bringing the water-containing fluid composition according to any one of claims 1 to 7 into contact with a target.

12. A processed lysozyme and/or a salt thereof having a fluorescence intensity, specified by the following definition, of 4000 or more:
the fluorescence intensity: a fluorescence intensity measured under conditions of an excitation wavelength of 390 nm (excitation bandwidth of 10 nm) and a fluorescence wavelength of 470 nm (fluorescence bandwidth of 10 nm), for a solution obtained by adding 25 µL of an 8 mM solution of 1,8-anilinonaphthalenesulfonic acid in methanol to 5 mL of a diluted solution obtained by diluting with a phosphate buffer solution (pH 7.0) to a concentration in terms of solid content of the processed lysozyme and/or the salt thereof of 0.05 mass% and a concentration of phosphate of 0.2 M, and thereafter allowing the solution to react at room temperature for 30 minutes.

13. The processed lysozyme and/or the salt thereof according to claim 12, wherein an anti-norovirus activity specified below is 2.0 or more:
the anti-norovirus activity: a value obtained by subtracting a logarithm of infectivity of a norovirus mixed solution obtained by mixing a norovirus solution with a 2 mass% aqueous solution of the processed lysozyme and/or the salt thereof in equal amounts after the norovirus mixed solution is allowed to stand at room temperature for one minute, from a logarithm of infectivity of the norovirus mixed solution before being allowed to stand.

14. A method for producing the processed lysozyme and/or the salt thereof according to claim 12 or 13, comprising:
a first heating step of heating an aqueous solution of the lysozyme and/or the salt thereof having a light transmittance at 660-nm wavelength of more than 70%, a pH of 5.0 or more and 7.0 or less, and a concentration in terms of solid content of the lysozyme and/or the salt thereof of 0.5 mass% or more and 7 mass% or less, until the light transmittance at 660-nm wavelength of the aqueous solution reaches 70%;
a second heating step of heating the aqueous solution after the first heating step until the light transmittance at 660-nm wavelength of the aqueous solution reaches a minimum value of less than 70% and thereafter heating the aqueous solution up to 70%; and
a third heating step of further heating the aqueous solution after the second heating step in a state where the light transmittance at 660-nm wavelength of the aqueous solution is more than 70%.

15. The method for producing the processed lysozyme and/or the salt thereof according to claim 14,
wherein the heating conditions in the third heating step are such that the heating is performed until the light transmittance at 660-nm wavelength of a mixture of a filtrate obtained by filtering the aqueous solution obtained in the third heating step through a 0.45 µm membrane filter with ethanol at a mass ratio of 1:1 reaches 85% or more.

16. The method for producing the processed lysozyme and/or the salt thereof according to claim 14 or 15, further comprising:
a step of obtaining the processed lysozyme and/or the salt thereof in powder form by spray drying or freeze drying the aqueous solution after the third heating step.

17. A method for producing a water-containing fluid composition, comprising:
a step of obtaining a water-containing fluid composition having a concentration of a polar organic solvent of 55 mass% or more and 90 mass% or less by mixing the processed lysozyme and/or the salt thereof obtained by the method according to any one of claims 14 to 16 with the polar organic solvent.

18. A method for producing a water-containing fluid composition, comprising:
a step of obtaining a water-containing fluid composition having a concentration of a polar organic solvent of 55 mass% or more and 90 mass% or less by mixing the processed lysozyme and/or salt thereof according to claim 12 or 13 with the polar organic solvent.

19. The method for producing a water-containing fluid composition according to claim 17 or 18,
wherein in the step of obtaining the water-containing fluid composition, the processed lysozyme and/or the salt thereof is mixed with the polar organic solvent so that a concentration in terms of solid content of the processed lysozyme and/or the salt thereof in the water-containing fluid composition is 0.1 mass% or more and 10 mass% or less.
